(19) Europäisches Patentamt
European Patent Office
Office européen des brevets

(11) Publication number: 0 265 020 B1

(12) EUROPEAN PATENT SPECIFICATION

(45) Date of publication of the patent specification: 29.08.90

(51) Int. Cl.⁵: C07D 307/87, A01N 43/12

(21) Application number: 87202009.4

(22) Date of filing: 19.10.87

# (54) Diphenyl ether herbicides.

(30) Priority: 20.10.86 GB 8625106

(43) Date of publication of application: 27.04.88 Bulletin 88/17

(45) Publication of the grant of the patent: 29.08.90 Bulletin 90/35

(84) Designated Contracting States: AT BE CH DE ES FR GB GR IT LI LU NL SE

(56) References cited:
EP-A- 0 145 078

CHEMICAL ABSTRACTS, vol. 107, no. 5 August 3, 1987, Columbus, Ohio, USA SHELL INTERNATIONALE RESEARCH MAATSCHAPPIJ B.V. "Preparation of phenoxyphthalide derivatives as herbicides" page 679, column 1, abstract-no. 39 597c
CHEMICAL ABSTRACTS, vol. 93, no. 13, September 29, 1980, Columbus, Ohio, USA HIRAGA, KUNIKAZU; SHIBAYAMA, SHOICHI; OKAWA, KATSUMASHA; HARADA, TATSUO "Phenoxyphtalates and herbicidal compositions containing them" page 639, column 2, abstract-no. 132 259wtegrated services" 000

The file contains technical information submitted after

(73) Proprietor: SHELL INTERNATIONALE RESEARCH MAATSCHAPPIJ B.V., Carel van Bylandtlaan 30, NL-2596 HR Den Haag(NL)

(72) Inventor: Clark, Michael Thomas, Torcross, Wises Lane Borden, Sittingbourne Kent(GB)
Inventor: Raven, Clive Alan, Hemsby House Hearts Delight, Borden Nr. Sittingbourne Kent(GB)
Inventor: Gilmore, Ian James, 32 Waterloo Road, Sittingbourne Kent(GB)

(74) Representative: Bennett, David Arthur Horder et al, 4, York Road, London SE1 7NA(GB)

(56) References cited: (continuation)
the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

This invention relates to certain diphenyl ether derivatives, the preparation of such compounds, herbicidal compositions containing them, and to their use in combating undesired plant growth.

The applicants' European Patent Application No. 145078 describes and claims a herbicidal composition which comprises a carrier and, as active ingredient, a diphenyl ether derivative having the general formula I:

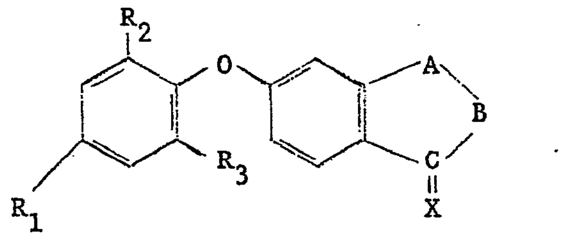

(I)

wherein $R_1$ represents a hydrogen or halogen atom or an alkyl or haloalkyl group; $R_2$ and $R_3$, which may be the same or different, each independently represents a hydrogen or halogen atom or an alkyl, haloalkyl, nitro or cyano group; and B and A, inter alia, represent, respectively, an oxygen atom and a group of formula $=C(R_4)_2$ in which each $R_4$, which may be the same or different, represents a hydrogen or halogen atom or an optionally substituted alkyl, cycloalkyl, alkenyl, alkynyl, aryl, aralkyl, alkaryl, alkoxy, cycloalkoxy, alkenyloxy, alkynyloxy, alkoxycarbonylalkoxy, alkylthio, acyl, acyloxy, carboxyl, alkoxycarbonyl or heterocyclic group, an amino group of formula $NR_6R_7$, or when one $R_4$ represents a hydrogen atom, an alkoxy group or a hydrocarbon group, then the other may represent a hydroxyl group, or both groups $R_4$ together may represent an imino group of formula $=NR_6$;
$R_5$ represents a hydrogen atom, a carboxyl or hydroxyl group, or an optionally substituted alkyl, alkoxy, aralkyl, alkoxycarbonyl or alkoxycarbonylalkyl group, or an amino group of formula $NR_6R_7$;
$R_6$ and $R_7$, which may be the same or different, each represents a hydrogen atom or an optionally substituted alkyl, aryl or acyl group;
and X represents an oxygen or sulphur atom.

It has now been found, unexpectedly, that a certain group of the compounds broadly described in EPA 145078, but not specifically disclosed therein, possess significantly higher selectivity in cereal crops than a representative diphenyl ether phthalide actually disclosed.

Accordingly, the present invention provides phenoxy phthalide derivatives having the general formula II:-

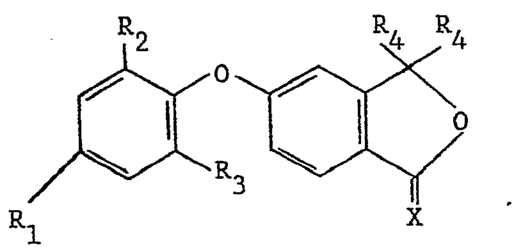

(II)

wherein $R_1$ represents a hydrogen or halogen, preferably chlorine, atom or an alkyl or haloalkyl group, suitably of 1-4 carbon atoms, preferably trifluoromethyl; $R_2$ $R_3$, which may be the same or different, each independently represents a hydrogen or halogen, preferably chlorine, atom or an alkyl or haloalkyl group, suitably of 1-4 carbon atoms, for example trifluoromethyl, or a nitro or cyano group; each group $R_4$ is the same and represents a $C_{2-6}$ alkenyl or alkynyl group; and X represents an oxygen or sulphur atom.

Preferred compounds are those wherein $R_1$ represents a halogen, more particularly chlorine, atom or, especially, a trifluoromethyl group;$R_2$ represents a nitro, cyano, trifluoromethyl or, especially, a halogen, more particulaly chlorine, atom; and $R_3$ represents a halogen, more particularly chlorine, or, especially, a hydrogen atom.The groups $R_4$ are identical groups having 2-6 carbon atoms, especially identical groups having 2-4 carbon atoms. More preferably still, the groups $R_4$ are selected from vinyl, allyl and ethynyl.

The invention also provides a process for the preparation of a phenoxy phthalide derivative of formula II as defined above which comprises reacting a compound of formula III

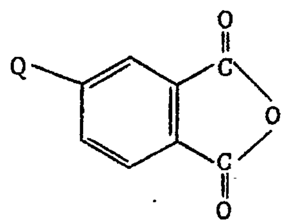

(III)

where Q represents a halogen atom or a nitro group or a group of formula -OZ, where Z represents a hydrogen or alkali metal atom or a group of formula

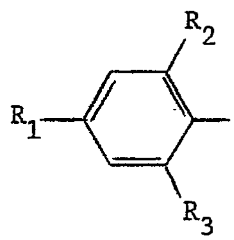

with a compound of formula $R^4$-M-Hal (IV) where M represents a metal atom and Hal represents a halogen atom; and, when the product is a compound in which Q does not represent a group of formula

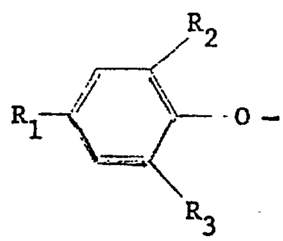

reacting said compound with a compound of formula V

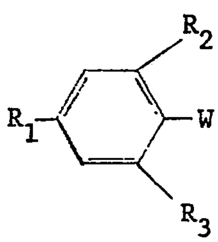

(V)

in which compound W represents a halogen atom or a nitro group when Q represents a group -OZ where Z represents a hydrogen or alkali metal atom, and W represents such a group -OZ when Q represents a halogen atom or a nitro group; and optionally converting a compound of formula II where X is an oxygen atom to a compound of formula II where X is a sulphur atom.

The moiety Hal is preferably a bromine or iodine atom.

Compounds wherein X represents sulphur may be obtained from the corresponding oxygen analogues by reaction with phosphorus pentasulphide.

The organometallic reagent used is preferably an organomagnesium compound (Grignard reagent), which may be prepared according to established procedures, e.g. by taking up the appropriate alkenyl or alkynyl halide and magnesium metal in an aliphatic ether, such as diethyl ether, in the absence of water, or may be prepared by passing the alkene or alkyne gas through a solution of an alkyl Grignard reagent. The reaction of the compound of formula III with that Grignard reagent is suitably carried out in a solvent, which may also be diethyl ether, or may be a different inert organic solvent such as tetrahydrofuran. The formation of the Grignard reagent and its reaction with the compound of formula III are each suitably carried out in the temperature range 0 to 50°C, preferably at ambient temperature. The Grignard organomagnesium complex may be supplemented by the generation of an organocadmium complex through the addition of cadmium chloride.

When the above reactions are effected to make compounds in which Q is other than a group

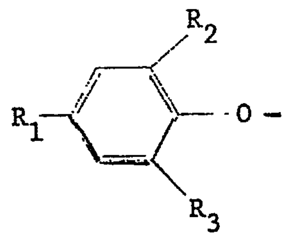

that is to say, compounds in which one of W and Q represents a halogen, suitably chlorine, atom or nitro group and the other represents a group of formula -OZ where Z represents a hydrogen atom or alkali metal atom, it is preferably the moiety W which represents the halogen atom or nitro group. The reaction is conveniently carried out by reacting a compound of formula V wherein W represents a halogen, suitably chlorine, atom or a nitro group with an alkali metal alkoxide compound of formula III produced by reacting the corresponding compound in which Q represents hydroxy, with an alkali metal hydroxide in an alkanol solvent, for example, ethanol. The reaction of the alkoxide with the compound V is preferably carried out in a suitable solvent, for example, dimethyl sulphoxide, sulpholane, dimethyl formamide, or dimethyl acetamide at elevated temperature, conveniently under reflux, and also under an inert atmosphere such as nitrogen.

It is preferred, however, to effect the above reactions with starting materials in which Q represents a group

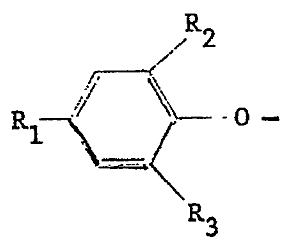

so that a further step to produce compounds of formula II is not required.

Since the phthalic anhydride starting material mentioned above contains two ring carbonyl groups, the Grignard reagent may react with either of them. In practice, it is usually found that the major product is the desired phthalide of formula II, with the isomeric phthalide of formula VI below being formed as a minor by-product

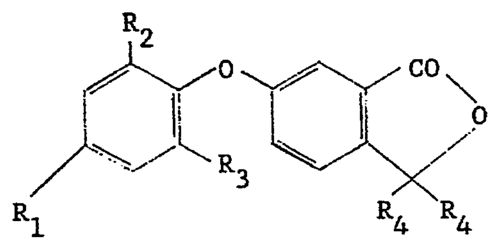

VI

The compounds of general formula II have been found to show interesting activity as herbicides, in particular in respect of their behaviour in selectively combating weeds in cereal crops such as wheat. Accordingly, the invention further provides a herbicidal composition comprising a compound of formula II as defined above in association with at least one carrier, and a method of making such a composition which comprises bringing a compound of formula II into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence. The dosage of active ingredient used may, for example, be from 0.01 to 10 kg/ha, preferably 0.01 to 4kg/ha. A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sul-phonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 -0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated by the following Examples.

Example 1

5-(2'-Chloro-4'trifluoromethylphenoxy)-3,3-divinyl phthalide

To 1M vinyl magnesium bromide in tetrahydrofuran (75ml) was added 50 ml of dry tetrahydrofuran, then, dropwise, 5-(2-chloro-4-trifluormethylphenoxy)phthalic anhydride (3.4g) dissolved in dry tetrahydrofuran (50ml). After addition was complete the mixture was stirred for ½ hour at ambient temperature,

then poured into an ice/concentrated hydrochloric acid mixture. The resulting solution was extracted with diethyl ether. The extracts were dried (sodium sulphate), and the product chromatographically purified on silica using dichloromethane as eluant. The title compound was the second fraction, a pale straw coloured oil (1.6g).

Analysis: Calculated :59.9%C; 3.15%H
Found :59.2%C; 3.4%H

N.M.R. results: delta 6.92-7.85 ppm multiplet (6H)
delta 6.07 ppm doublet of doublets (2H)
delta 5.38 ppm doublet of doublets (4H)

Example 2

5-(2'-Chloro-4'-trifluoromethylphenoxy)-3,3-diallylphthali e

1M allyl magnesium bromide in ether (50 ml) was syringed into a flask containing dry tetrahydrofuran (30ml). 5-(2-Chloro-4-trifluoromethylphenoxy)phthalic anhydride (2.3g) dissolved in dry tetrahydrofuran (30ml) was added dropwise with stirring at ambient temperature, under dry nitrogen. After addition was complete the mixture was stirred for ¼ hr at ambient temperature, poured onto ice/concentrated hydrochloric acid, and extracted with diethyl ether (4×100 ml). The ether extracts were washed, dried (sodium sulphate), and chromatographically purified on silica, using dichloromethane as eluant, to yield the title compound (second fraction, pale straw coloured oil, 1.0g).

Analysis: Calculated:61.7%C 3.9%H
Found:61.0%C 4.0%H

N.M.R. results: delta 6.85 - 7.82 ppm multiplet (6H)
delta 5.55 multiplet (2H)
delta 5.08 doublet of doublets (4H)
delta 2.65 doublet of quartets (4H)

Example 3

5-(2'-Chloro-4'-trifluoromethylphenoxy)-3,3-diethynyl phthalide

Dry ethyl bromide (8.5g) was added dropwise to a suspension of magnesium metal turnings (1.8g) in dry tetrahydrofuran (75ml), under dry nitrogen. The mixture was stirred until the magnesium had dissolved. Dry acetylene was bubbled into the mixture for 20 minutes until ethane was no longer produced, whilst the temperature was maintained between 25 and 40°C. 5-(2-Chloro-4-trifluoromethylphenoxy)phthalic anhydride (3.1g) in dry tetrahydrofuran (60ml) was added dropwise to the Grignard reagent over 1 hour, under dry nitrogen. ¼ hour after addition the mixture was poured onto ice/10% sulphuric acid. The mixture was extracted with dichloromethane (5×100ml), washed, dried (sodium sulphate), and the solvent removed by evaporation. The resulting brown oil was purified by chromatography on silica using dichloromethane as eluant. The title compound, the second fraction, was a pale yellow solid (1.1g; mp 154-6°C). Rechromatography of the remaining fractions yielded a further 0.3g of the title compound.

Analysis: Calculated:60.6%C 2.1%H
Found:60.8%C 2.4%H

NMR results: delta 7.13-7.86 ppm multiplet (6H)
delta 2.80 ppm singlet (2H)

Example 4

Herbicidal Activity

To evaluate their herbicidal activity, compounds according to the invention were tested using as representative range of plants: maize, Zeamays (Mz); rice, Oryzasativa (R); barnyard grass, Echinochloacrusgalli (BG); oat, Avenasativa (O); linseed, Linumusitatissimum (L); mustard, Sinapsisalba (M); sugar beet, Betavulgaris (SB) and soya bean, Glycinemax (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels corresponding to 5 kg or 1 kg of active material per hectare in a volume equivalent to 900 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table 1 below, in which the compounds are identified by reference to the preceding examples.

TABLE 1

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 3 | 2 | 6 | 7 | 6 | 4 | 7 | 4 | 5 | 7 | 4 | 9 | 9 | 9 | 9 | 9 | 9 | 4 | 3 | 8 | 6 | 8 | 9 | 9 | 4 |
| | | | | | | | | | 1 | 4 | 2 | 9 | 8 | 9 | 9 | 9 | 9 | 1 | 1 | 7 | 4 | 6 | 8 | 8 | 1 |
| 2 | 0 | 0 | 0 | 0 | 0 | 0 | 0 | 0 | 5 | 6 | 3 | 8 | 5 | 8 | 7 | 9 | 6 | 0 | 0 | 2 | 2 | 3 | 5 | 8 | 0 |
| | | | | | | | | | 1 | 5 | 1 | 7 | 3 | 6 | 6 | 7 | 6 | 0 | 0 | 1 | 1 | 3 | 4 | 4 | 0 |
| 3 | 0 | 0 | 2 | 3 | 4 | 0 | 0 | 0 | 5 | 6 | 5 | 9 | 7 | 9 | 9 | 9 | 9 | 0 | 0 | 7 | 2 | 9 | 9 | 9 | 2 |
| | | | | | | | | | 1 | 4 | 3 | 8 | 6 | 9 | 9 | 9 | 9 | 0 | 0 | 7 | 1 | 8 | 8 | 8 | 2 |

Example 5

Herbicidal Activity

Further biological evaluations were carried with the compounds of Examples 1 and 3, which compared their properties with those of 5-(2'-chloro-4'-trifluoromethylphenoxy)phthalide, (hereinafter designated as "A") specifically described as Example 1 of EP-A-145078.

The tests conducted were spray tests, in which seedling plants of a range of species were sprayed with the test compounds. The test plant species were wheat (WH), chickweed (ST), mayweed (MW), pale persicaria (PP), field forget-me-not (FF), speedwell (SW), field pansy (FP), cleavers (GG), sugar beet (SB) and oil-seed rape (RA). The plants were at the stage of 1-3 true leaves.

The soil used in the test was a prepared horticultural loam.

The compounds were tested as technical materials and formulated in a 1:1 acetone:water mix containing up to 0.2% of the alkylphenol/ethylene oxide wetting agent, Triton X155 (trade mark) and applied as single dose foliar sprays in a total volume of 600 litres/hectare. Application was at 4 different dosage levels 0.3, 0.1, 0.03 and 0.01 kg/ha designed to produce a range of responses. At least two replicate pots were used for each treatment. Untreated seedling plants were used as controls.

Phytotoxicity compared with the untreated control was assessed visually approximately 2 weeks after treatment using the standard 0-9 scale, 0 indicating no effect and 9 indicating death.

The results were subjected to a standard probit analysis by computer to calculate the dosage of each compound in g/ha required to kill 50% of the weed species and to produce 50% level of effect on the crop species. These dosages are referred to as the $GID_{50}$ value.

The $GID_{50}$ values from two sets of tests (test 1, comparing the compounds of Examples 1 and 3 with A, test 2, comparing the compound of Example 1 with A) are set out below in Tables 2 and 3.

Table 2 (Test 1)

| $GID_{50}$ values expressed in g/ha | | | | | |
|---|---|---|---|---|---|
| Test Compound | WH | GG | SW | SB | RA |
| Example 1 | 31 | 1 | 1 | 4 | 1 |
| Example 3 | 75 | 5 | 5 | 10 | 4 |
| A | 64 | 7 | 13 | 43 | 27 |

Table 3 (Test 2)

| $GID_{50}$ values expressed in g/ha | | | | | | | | |
|---|---|---|---|---|---|---|---|---|
| Test Compound | WH | GG | SW | SB | MW | FP | PP | FF |
| Example 1 | 38 | 3 | 4 | 1 | 1 | 1 | 2 | 2 |
| A | 63 | 22 | 14 | 44 | 87 | 4 | 4 | 13 |

These $GID_{50}$ values were then used to calculate the selectivity factors in wheat by dividing the $GID_{50}$ value of the compounds in wheat by their $GID_{50}$ value in each weed species. (NB. Numbers greater than 1 indicate selectivity between crop and weed and the larger the number the greater the selectivity).

The results are set out in Tables 4 and 5 below.

Table 4 (test 1)

| Selectivity Factors | | | | | |
|---|---|---|---|---|---|
| Test Compound | GG | SW | SB | RA | Mean value |
| Example 1 | 31.0 | 31.0 | 7.8 | 31.0 | 25.2 |
| Example 3 | 15.0 | 15.0 | 7.5 | 18.8 | 14.1 |
| A | 9.1 | 4.9 | 1.5 | 2.3 | 4.5 |

Table 5 (test 2)

| Selectivity Factors | | | | | | | | |
|---|---|---|---|---|---|---|---|---|
| Test Compound | GG | SW | SB | MW | FP | PP | FF | Mean value |
| Example 1 | 12.7 | 9.5 | 38.0 | 38.0 | 38.0 | 19.0 | 19.0 | 24.9 |
| A | 2.9 | 4.5 | 1.4 | 0.7 | 15.8 | 15.8 | 4.8 | 6.6 |

## Claims

1. A phenoxy phthalide derivative of the general formula II:

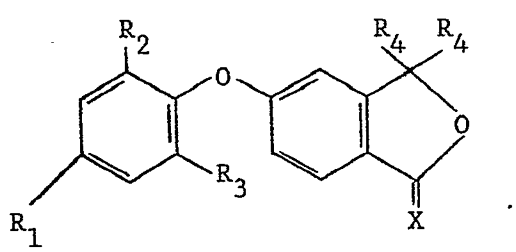

(II)

wherein $R_1$ represents a hydrogen or halogen atom or a $C_{1-4}$ alkyl or haloalkyl group; $R_2$ and $R_3$, which may be the same or different, each independently represents a hydrogen or halogen atom or a $C_{1-4}$ alkyl, $C_{1-4}$ haloalkyl, nitro or cyano group; each group $R_4$ is the same and represents a $C_{2-6}$ alkenyl or $C_{2-6}$ alkynyl group; and X represents an oxygen or sulphur atom.

2. A compound as claimed in claim 1 wherein X represents an oxygen atom.

3. A compound as claimed in claim 1 or 2 wherein $R_1$ represents a trifluormethyl group; $R_2$ represents a halogen atom; and $R_3$ represents a hydrogen atom.

4. A compound as claimed in claim 1, 2 or 3 wherein both groups $R_4$ represent a vinyl group, an allyl group or an ethynyl group.

5. A process for the preparation of a phenoxy phthalide derivative of formula II as claimed in any one of claims 1 to 4, which comprises reacting a compound of formula III

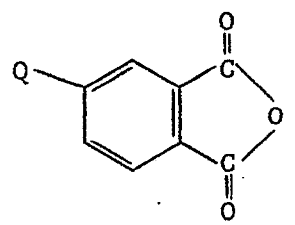

(III)

where Q represents a halogen atom or a nitro group or a group of formula -OZ, where Z represents a hydrogen or alkali metal atom or a group of formula

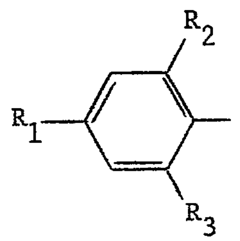

with a compound of formula $R^4$-M-Hal (IV) where M represents a metal atom and Hal represents a halogen atom; and, when the product is a compound in which Q does not represent a group of formula

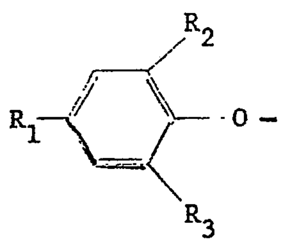

reacting said compound with a compound of formula V.

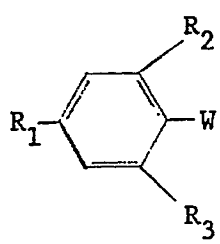

(V)

in which compound W represents a halogen atom or a nitro group when Q represents a group -OZ where Z represents a hydrogen or alkali metal atom, and W represents such a group -OZ when Q represents a halogen atom or a nitro group; and optionally converting a compound of formula II where X is an oxygen atom to a compound of formula II where X is a sulphur atom.

6. A herbicidal composition, which comprises a compound as claimed in any one of claims 1 to 4, together with a carrier.

7. A method of combating undesired plant growth at a locus, which comprises treating the locus with a compound as claimed in any one of claims 1 to 4, or a composition as claimed in claim 6.

8. The use of a compound as claimed in any one of claims 1 to 4, as a herbicide.

## Patentansprüche

1. Phenoxyphtalidderivat der allgemeinen Formel II:

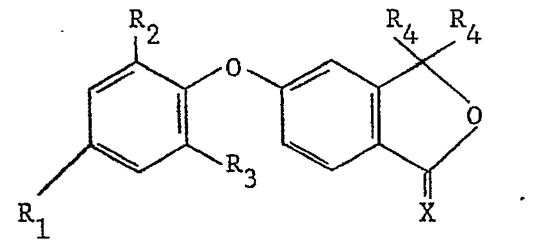

(II)

worin
$R_1$ ein Wasserstoff- oder Halogenatom oder eine $C_{1-4}$-Alkyl-oder Halogenalkylgruppe darstellt;
$R_2$ und $R_3$, die gleich oder verschieden sein können, jeweils unabhängig voneinander ein Wasserstoff- oder Halogenatom oder eine $C_{1-4}$-Alkyl-, $C_{1-4}$-Halogenalkyl-, Nitro- oder Cyanogruppe bedeuten; jede Gruppe $R_4$ die gleiche Bedeutung hat und eine $C_{2-6}$ Alkenyl- oder $C_{2-6}$ Alkynylgruppe darstellt; und
X ein Sauerstoff- oder Schwefelatom bedeutet.

2. Verbindung nach Anspruch 1, worin X ein Sauerstoffatom bedeutet.

3. Verbindung nach Anspruch 1 oder 2, worin
$R_1$ ein Trifluormethylgruppe darstellt;
$R_2$ ein Halogenatom bedeutet; und
$R_3$ ein Wasserstoffatom darstellt.

4. Verbindung nach Anspruch 1, 2 oder 3, worin beide Gruppen $R_4$ eine Vinylgruppe, eine Allylgruppe oder eine Ethinylgruppe bedeuten.

5. Verfahren zur Herstellung eines Phenoxyphtalidderivats der Formel II, wie in einem der Ansprüche 1 bis 4 beansprucht, welches ein Umsetzen einer Verbindung der Formel III

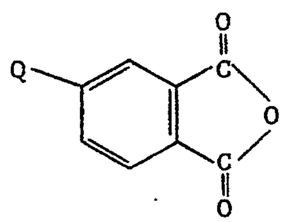

(III)

worin Q ein Halogenatom oder eine Nitrogruppe oder eine Gruppe der Formel -OZ, worin Z ein Wasserstoff- oder Alkalimetallatom oder eine Gruppe der Formel

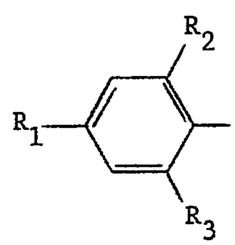

bedeutet, mit einer Verbindung der Formelo R-M-Hal (IV) umfaßt, worin M ein Metallatom bedeutet und Hal ein Halogenatom darstellt; und, falls das Produkt eine Verbindung ist, worin Q nicht eine Gruppe der Formel

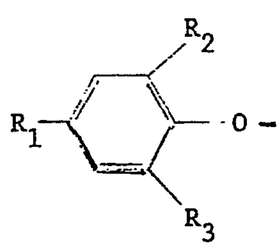

bedeutet, ein Umsetzen dieser Verbindung mit einer Verbindung der Formel V

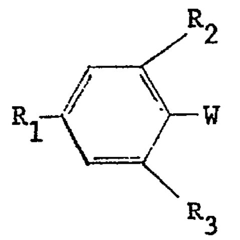

(V)

in welcher Verbindung W ein Halogenatom oder eine Nitrogruppe darstellt, wenn Q eine Gruppe -OZ bedeutet, worin Z ein Wasserstoff- oder Alkalimetallatom darstellt, und W eine solche Gruppe -OZ darstellt, wenn Q ein Halogenatom oder eine Nitrogruppe bedeutet; und gegebenenfalls Überführen einer Verbindung der Formel II, worin X ein Sauerstoffatom darstellt in eine Verbindung der Formel II, worin X ein Schwefelatom bedeutet.

6. Herbizide Zusammensetzung, welche eine Verbindung, wie in einem der Ansprüche 1 bis 4 beansprucht, zusammen mit einem Träger umfaßt.

7. Verfahren zur Bekämpfung von unerwünschtem Pflanzenwuchs an einem Ort, welches ein Behandeln des Ortes mit einer Verbindung, wie in einem der Ansprüche 1 bis 4 beansprucht, oder mit einer Zusammensetzung, wie in Anspruch 6 beansprucht, umfaßt.

8. Verwendung einer Verbindung, wie in einem der Ansprüche 1 bis 4 beansprucht, als Herbizid.

**Revendications**

1. Un dérivé de phénoxy phtalide de la formule générale II:

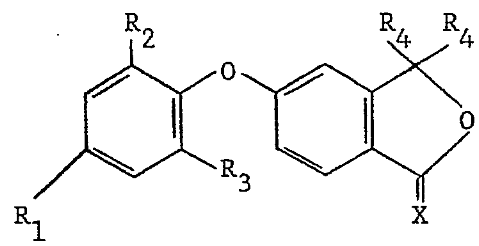

(II)

où $R_1$ représente un atome d'hydrogène ou d'un halogène ou un groupe $C_{1-4}$ alcoyle ou haloalcoyle; $R_2$ et $R_3$, qui peuvent être identiques ou différentes, représentent chacun indépendamment un atome d'hydrogène ou d'un halogène ou un groupe $C_{1-4}$ alcoyle, $C_{1-4}$ haloalcoyle, nitro ou cyano; les groupes $R_4$ sont identiques et représentent chacun un groupe $C_{2-6}$ alcényle ou $C_{2-6}$ alcynyle; et X représente un atome d'oxygène ou de soufre.

2. Un composé selon la revendication 1, dans lequel X représente un atome d' oxygène.

3. Un composé selon la revendication 1 ou 2, dans lequel $R_1$ représente un groupe trifluorométhyle; $R_2$ représente un atome d'halogène; et $R_3$ représente un atome d'hydrogène.

4. Un composé selon la revendication 1, 2 ou 3, dans lequel les deux groupes $R_4$ représentent chacun un groupe vinyle, un groupe allyle ou un groupe éthynyle.

5. Un procédé pour la préparation d'un dérivé de phénoxy phtalide de formule II tel que défini dans l'une quelconque des revendications 1 à 4, qui comprend la réaction d'un composé de formule III.

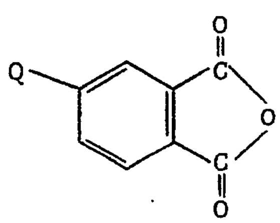

(III)

où Q représente un atome d'halogène ou un groupe nitro ou un groupe de formule -OZ, où Z représente un atome d'hydrogène ou d'un métal alcalin ou un groupe de formule

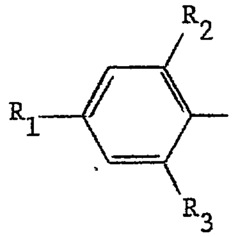

avec un composé de formule $R^4$-M-Hal (IV), où M représente un atome de métal et Hal représente un atome d'halogène; et, quand le produit est un composé dans lequel Q ne représente pas un groupe de formule

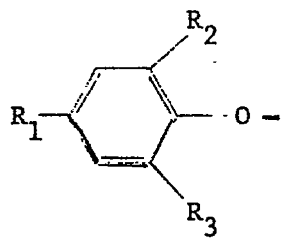

la réaction de ce composé avec un composé de formule V

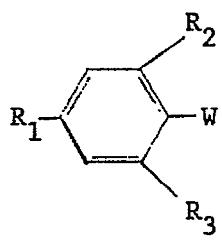

(V)

composé dans lequel W représente un atome d'halogène ou un groupe nitro quand Q représente -OZ où Z représente un atome d'hydrogène ou d'un métal alcalin, et W représente un tel groupe -OZ quand Q représente un atome d'halogène ou un groupe nitro; et éventuellement la conversion d'un composé de formule II dans lequel X est un atome de soufre.

6. Une composition herbicide, qui comprend un composé selon l'une quelconque des revendications 1 à 4 en même temps qu'un véhicule.

7. Une méthode de lutte contre la croissance de plantes indésirables en un lieu, qui comprend le traitement du lieu avec un composé selon l'une quelconque des revendications 1 à 4 ou une composition selon la revendication 6.

8. L'utilisation d'un composé selon l'une quelconque des revendications 1 à 4 comme herbicide.